## Europäisches Patentamt

## European Patent Office

## Office européen des brevets

(11) Veröffentlichungsnummer: **0 263 426**
**A2**

# EUROPÄISCHE PATENTANMELDUNG

(21) Anmeldenummer: **87114246.9**

(22) Anmeldetag: **30.09.87**

(51) Int. Cl.4 **G01N 25/56** , G01N 25/70 , G01N 1/06 , G01N 33/34 , G03C 1/76

(30) Priorität: **10.10.86 DE 3634518**

(43) Veröffentlichungstag der Anmeldung:
**13.04.88 Patentblatt 88/15**

(84) Benannte Vertragsstaaten:
**BE DE FR GB NL**

(71) Anmelder: **Agfa-Gevaert AG**
**Patentabteilung**
**D-5090 Leverkusen 1(DE)**

(72) Erfinder: **Hamed, Saleman**
**Am Büscherhof 15**
**D-5653 Leichlingen 1(DE)**
Erfinder: **Söder, Jörg Michael, Dr.**
**Rene-Bohn-Strasse 22**
**D-5000 Köln 80(DE)**

(54) **Verfahren und Vorrichtung zur Messung der Gleichgewichtsfeuchte in einem hygroskopischen, flächenhaften, beidseitig beschichteten Material.**

(57) In einem Verfahren zur Messung der Gleichgewichtsfeuchte in hygroskopischem, flächenhaftem, beidseitig beschichtetem Material, vorzugsweise in beidseitig beschichtetem Rohpapier der Unterlage eines fotografischen Papiers, durch Bestimmung der relativen Feuchte der mit dem Material in Berührung stehenden Luft werden Proben dem Material unter Bildung von Schnittflächen entnommen und in einem Behälter eingebracht, der gegenüber der Außenluft abschließbar ist, wobei nach Einstellung der Gleichgewichtsfeuchte in diesem Behälter die relative Feuchte der darin enthalteren Luft gemessen wird.

EP 0 263 426 A2

# Verfahren und Vorrichtung zur Messung der Gleichgewichtsfeuchte in einem hygroskopischen, flächenhaften, beidseitig beschichteten Material

Die Erfindung betrifft ein Verfahren zur Messung der Gleichgewichtsfeuchte in einem hygroskopischen, flächenhaften, beidseitig beschichteten Material, vorzugsweise in der Unterlage eines fotografischen Papiers, durch Ermittlung der relativen Feuchte der mit dem Material im Feuchtigkeitsaustausch stehenden Umgebungsluft. Ferner betrifft die Erfindung eine Vorrichtung zur Durchführung dieses Verfahrens.

Für Color-und Schwarz/Weiß-Fotopapiere werden als Unterlagen für die fotografisch wirksamen Emulsionsschichten meist Rohpapiere mit einem Flächengewicht von ca. 50 bis 300 $g/m^2$ verwendet. Dabei weist das Rohpapier eine Dicke von ca. 80 bis 130 $\mu$m auf. Dieses Rohpapier wird beidseitig mit einem Polymeren, vorzugsweise Polyethylen, von 20 bis 40 $\mu$m Dicke beschichtet. Diese Oberflächenschichten der Unterlage verhindern eine rasche Wasseraufnahme des Rohpapiers beim späteren Entwicklungsprozeß nach der fotografischen Belichtung des Fotopapiers.

Bei der Herstellung des fotografischen Papiers werden die fotografisch wirksamen Emulsionsschichten auf die vorerwähnte Unterlage aufgegossen, wobei die unterste Schicht des aufzugießenden Schichtpaketes mit der oberen Schichtfläche der Papierunterlage in Kontakt kommt. Das Schichtpaket wird nach dem Aufgießen auf die Unterlage erstarrt und die begossene Bahn auf bestimmte, vorgegebene Feuchtewerte getrocknet, bevor sie auf eine Aufwickelrolle aufgewickelt und für die spätere Konfektionierung bereitgestellt wird.

Eine zu hohe Feuchte kann zum Verkleben der einzelnen Windungen der aufgewickelten Bahn führen. Dies kann Anlaß sein für mechanische Beschädigungen oder elektrostatische Aufladungen und Verblitzungen. Schon geringfügig zu niedrige Feuchten können ebenfalls zu Aufladungen und Verblitzungen führen. Darüber hinaus werden die fotografischen Eigenschaften, insbesondere die Sensitometrie des Fotopapiers und dessen Haltbarkeit, vom Wassergehalt in starkem Maße beeinflußt.

Für den Wassergehalt des Fotopapiers spielt auch die Feuchte in der Papierseele der Unterlage eine wesentliche Rolle, zumal auch die Polyethylenbeschichtung einen Feuchtigkeitsaustausch zwischen der Papierseele, der Unterlage und dem hierauf befindlichen Schichtpaket bei längeren Lagerungszeiten nicht völlig verhindert.

Im einzelnen ist von folgenden Zusammenhängen auszugehen.

Entscheidend für die fotografischen Eigenschaften des Fotopapiers ist zunächst die Feuchte im Schichtpaket der Emulsionsschichten, das nur wenige $\mu$m dick ist. Bei der Produktion der Fotopapiere ist der Erstarrungs-und Trocknungsprozeß in den Papierbegießmaschinen darauf abgestellt, die fotografischen Schichten auf eine bestimmte Gleichgewichtsfeuchte im Bereich von 40 bis 60% r.F. zu trocknen. Nun stellt sich aber im fertigen, auf Rollen aufgewickelten Fotopapier insbesondere bei längerer Lagerung ein Feuchtegleichgewicht zwischen der Unterlage und dem Schichtpaket der Emulsionsschichten ein, da bei unterschiedlichen Feuchtewerten ein langsamer Ausgleich durch Diffusion von Wasser durch die Polyethylenschicht der Unterlage stattfindet. Dieses Gleichgewicht läßt sich je nach Dicke der Polyethylenschicht erst nach Tagen oder Wochen einstellen. Der Wassergehalt des vergleichsweise dicken Rohpapiers, welches die Seele der Unterlage darstellt, ist mit 4 bis 8 % meist wesentlich größer als der Wassergehalt der Emulsionsschichten. Deshalb soll auch der Wassergehalt des Rohpapiers der gewünschten Feuchte des Endproduktes angepaßt sein.

Um dies zu gewährleisten, ist es bei der Produktion der Fotopapiere zur Produktionskontrolle und Qualitätssicherung wichtig und wünschenswert, den Wassergehalt des beschichteten Rohpapiers der Unterlage zu messen.

Dies kann dadurch erfolgen, daß die Gleichgewichtsfeuchte im Rohpapier der Unterlage ermittelt wird. Dabei ist als Gleichgewichtsfeuchte diejenige Feuchte zu verstehen, welche sich mit dem Rohpapier als hygroskopischem Material bei konstanter Temperatur in einem abgeschlossenen System als Feuchtegleichgewicht einstellt. Bekanntlich stellt die Sorptionsisoterme im Gleichgewichtszustand die Beziehung zwischen dem absoluten Wassergehalt des hygroskopischen Materials und der Gleichgewichtsfeuchte dar, so daß über die Sorptionsisoterme aus dem gemessenen Wert der Gleichgewichtsfeuchte der absolute Wassergehalt im hygroskopischen Material errechnet werden kann. Hierzu wird beispielsweise verwiesen auf Stefan Gal: "Die Methodik der Wasserdampf-Sorptionsmessungen", Springer-Verlag, 1967.

Zur Messung der Gleichgewichtsfeuchte im Rohpapier der Unterlage für fotografische Papiere ist es bereits bekannt, Aufsetzfühler zu verwenden, die auf das Rohpapier hermetisch dicht aufgesetzt werden. Sie messen mit einem Luftfeuchtefühler die sich nach Ablauf eines Zeitraumes einstellende Gleichgewichtsfeuchte im abgeschlossenen Luftvolumen über dem Papier. Dieses Meßverfahren ist jedoch bei Unterlagen, die beidseitig mit Polyethylen beschichtet sind, nicht ohne weiteres zu verwenden, da die Diffusion von Wasser aus dem Rohpapier durch die Polyethylenschicht zu viel Zeit beansprucht, bis es zum Gleichgewichtszustand unter dem auf die Polyethylenschicht aufgesetzten Meßfühler kommt.

Zur Vermeidung dieses Nachteils wurde bei dickeren Unterlagen schon der Weg beschritten, eine der Polyethylenschichten vom Rohpapier stellenweise abzureißen, wodurch es möglich wird, den Aufsetzfühler direkt auf das Rohpapier aufzusetzen. Dieses Verfahren hat jedoch den Nachteil, daß beim stellenweisen Abreißen der Polyethylenschicht vom Rohpapier schon in kürzester Zeit eine erhebliche Feuchteänderung im Rohpapier durch Wasserdiffusion mit der Umgebungsluft eintritt. Dieses Verfahren erfordert deshalb erhebliche Sorgfalt und erfahrenes Meßpersonal. Außerdem lassen sich Unterlagen, deren Rohpapier ein Flächengewicht von ca. 120 g/m² unterschreitet, nicht mehr ohne Zerstörung des Rohpapiers aufreißen, so daß eine sichere Messung nicht mehr möglich ist.

Es wurde deshalb auch schon in Betracht gezogen, den Feuchtigkeitsgehalt des Rohpapiers der Unterlage dadurch zu ermitteln, daß die Unterlage ausgetrocknet und die sich durch den Feuchtigkeitsverlust ergebende Gewichtsdifferenz gemessen wird. Dieses Verfahren erfordert jedoch eine Zeit von mehreren Stunden und ist deshalb in der Praxis bei ber Produktionskontrolle und Qualitätssicherung nicht anwendbar.

Hier setzt die Erfindung ein. Ihr liegt die Aufgabe zugrunde, ein Verfahren zu schaffen, mit dem die Feuchte des Rohpapiers einer beidseitig beschichteten Unterlage unabhängig von der Dicke des Rohpapiers, also auch bei Unterlagen mit extrem dünner Papierseele, in einfacher Weise, aber dennoch sicher und in relativ kurzer Zeit bestimmt werden kann.

Diese Aufgabe wird durch das Verfahren mit den Merkmalen des Anspruchs 1 der beigefügten Patentansprüche gelöst. Weitere Ausgestaltungen der Erfindung ergeben sich aus den nachgeordneten Ansprüchen.

Das erfindungsgemäße Verfahren weist den Vorteil auf, daß es innerhalb kurzer Zeit sichere Meßwerte zu liefern vermag. Dies beruht darauf, daß die Feuchtigkeit, die im Material zwischen den Oberflächenbeschichtungen enthalten ist, durch die Schnittflächen der aus dem Material gewonnenen Proben relativ rasch herausdiffundieren kann, so daß sich im abgeschlossenen Behälter, der die Materialproben enthält, auch relativ rasch ein Feuchtegleichgewicht einstellen kann. Von weiterem Vorteil ist dabei, daß das Innere der Materialproben infolge der Oberflächenbeschichtungen mit der Umgebungsluft praktisch nicht in Berührung kommt.

Das Feuchtegleichgewicht im Behälter stellt sich umso rascher ein, je höher der Wert des Verhältnisses der Gesamtsumme aller Schnittflächen der in den Behälter eingebrachten Materialproben zum Volumen des Behälters ist. Versuche haben gezeigt, daß bei einem Behältervolumen von ca. 100 ml und bei Einbringung von etwa 30 bis 50 Materialproben, die als - scheibenförmige Ausstanzungen von ca. 5 mm Durchmesser aus dem Material ge wonnen werden, innerhalb von ca. 5 Minuten ein Feuchte-Gleichgewichtszustand im Behälter erreicht werden kann. Als Material wurde hierbei eine Unterlage für Fotopapiere benutzt, die aus polyethylenbeschichtetem Rohpapier besteht. Die Gleichgewichtsfeuchte stellte sich hierbei im Behälter bei ca. 50 % relativer Feuchtigkeit der Behälterluft ein. Bei diesen Gegebenheiten war der Einfluß der ursprünglich, d.h. vor Einbringung der Materialproben, im Behälterinneren vorhandenen Luftfeuchtigkeit vernachlässigbar.

Das erfindungsgemäße Verfahren kann aufgrund seiner Einfachheit auch von weniger geschultem Laborpersonal durchgeführt werden, ohne daß die Genauigkeit der erzielten Meßergebnisse darunter leidet.

Ein Ausführungsbeispiel der Erfindung ist in der nachfolgenden Beschreibung und in den beigefügten Zeichnungen näher dargestellt und erläutert. In den Zeichnungen zeigen.

Fig. 1 einen Querschnitt durch die Unterlage eines Fotopapiers in stark vergrößerter Darstellung,

Fig. 2 eine Vorrichtung zum Ausstanzen von Materialproben aus der Unterlage gemäß Fig. 1 und zum Sammeln dieser Proben in einem Behälter,

Fig. 3 eine Meßvorrichtung mit einem Behälter gemäß Fig. 2 in der Draufsicht und

Fig. 4 einen Schnitt durch Behälter und Meßfühler mit angeschlossener Anzeigevorrichtung.

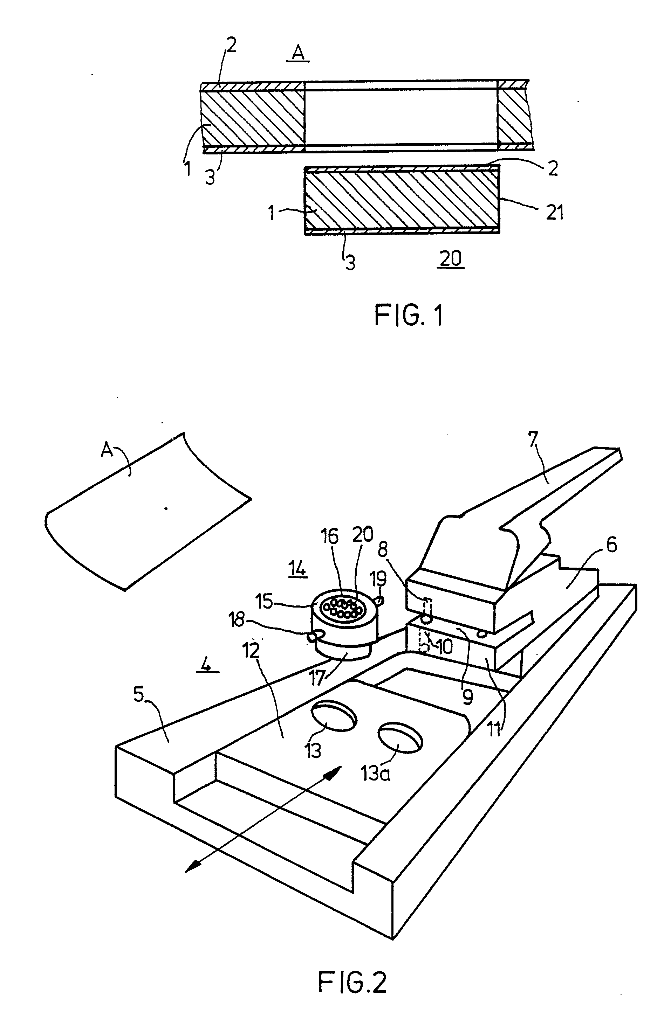

In Fig. 1 ist ein Ausschnitt aus einer flächenhaften Unterlage A für fotografisches Papier im Querschnitt stark vergrößert dargestellt. Sie besteht aus einer durch Rohpapier gebildeten flächenhaften Seele 1, deren obere und untere Flächen mit Schichten 2, 3 aus Polyethylen versehen sind.

Die in Fig. 2 dargestellte Stanzvorrichtung 4 umfaßt einen Rahmen 5, auf dem eine Stanze 6 einer an sich bekannten Bauart montiert ist. Diese besitzt einen handbedienten Schwenkhebel 7, durch welchen über ein nicht dargestelltes Getriebe ein Stanzstempel 8 senkrecht zu seiner Längsachse bewegbar ist und bei einer Abwärtsbewegung des Schwenkhebels 7 durch einen Materialeingabeschlitz 9 hindurch in eine Bohrung 10 im Fußteil 11 der Stanze eingreift. Die Bohrung 10 durchläuft die gesamte Wandstärke des überstehenden Fußteiles 11 und ist somit nach unten offen.

Im Rahmen 5 der Stanzvorrichtung 4 ist ein Schlitten 12 beweglich angeordnet. Er besitzt eine Öffnung 13, in welche ein Probenbehälter 14 einsetzbar ist. Es können im Schlitten 12 zusätzliche Aufnahmeöffnungen 13a für zusätzliche Probenbehälter vorgesehen sein, falls die Stanzvorrichtung 4 zusätzliche Stanzstempel aufweist.

Der Probenbehälter 14 besitzt eine ringförmige Wand 15, welche einen Innenraum 16 bildet, ein Fußteil 17 sowie diametral abstehende Lagerzapfen 18, 19. Der Behälter 14 ist mit seinem Fußteil 17 in die Öffnung 13 des Schlittens 12 der Stanzvorrichtung einsetzbar.

Zum Ausstanzen von Materialproben aus der Unterlage A wird in der Stanzvorrichtung 4 der zunächst noch leere Probenbehälter 14 in die Öffnung 13 des Schlittens 12 eingesetzt und mit dem Schlitten 12 unter die Bohrung 10 der Stanze 6 verfahren. Danach wird ein Materialstück der Unterlage A in den Schlitz 9 der Stanze 6 eingeführt. Durch wiederholtes Betätigen des Schwenkhebels 7 und gleichzeitiges Verschieben der Unterlage A im Schlitz 9 werden in entsprechender Anzahl kreisförmige Scheibchen 20 aus der Unterlage A ausgestanzt. Eine dieser - scheibchenförmigen Materialproben 20 mit zylindermantelförmiger Schnittfläche 21 ist in Fig 1 stark vergrößert dargestellt.

Die scheibchenförmigen Materialproben 20 fallen als Ausstanzungen aus dem in den Schlitz 9 eingeführten Materialstück der Unterlage A durch die Bohrung 10 nacheinander in den Innenraum 16 des Probenbehälters 14 und werden dort in loser Schichtung gesammelt.

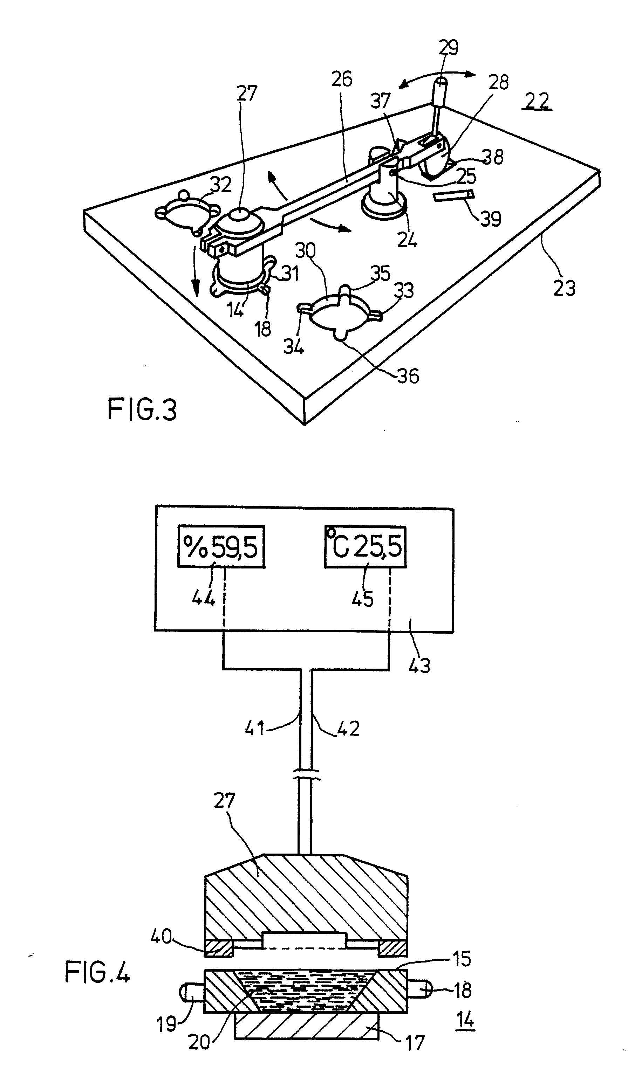

Nachdem eine hinreichende Anzahl von ausgestanzten Materialproben 20 gewonnen und im Probenbehälter 14 aufgefangen wurde, wird der Probenbehälter 14 nach Zurückfahren des Schlittens 12 der Stanzvorrichtung entnommen und in die in Fig. 3 dargestellte Meßvorrichtung 22 eingesetzt.

Die Meßvorrichtung 22 besitzt eine Grundplatte 23, auf der eine Säule 24 um ihre Längsachse drehbar gelagert ist. Auf der Säule 24 ist um die Achse 25 ein zweiarmiger Hebel 26 drehbar gelagert. Dieser trägt am einen Ende in fester Einspannung einen Feuchte-Meßfühler 27 und am anderen Ende einen Exzenter 28 mit einem Betätigungshebel 29.

Aufgrund der drehbaren Lagerung der Säule 24 und des Hebels 26 ist der Meßfühler 27 in zwei Freiheitsgraden schwenkbar, und zwar sowohl parallel zur Grundplatte 23 als auch in Ebenen senkrecht zu dieser Grundplatte.

Im horizontalen Schwenkbereich des Meßfühlers 27 sind drei kreisrunde Ausnehmungen 30, 31, 32 vorgesehen. Jede dieser Ausnehmungen enthält Lagermulden 33, 34 und Griffmulden 35, 36. Der Probenbehälter 11 kann nach Entnahme aus der Stanzvorrichtung 4 wahlweise in eine der Ausnehmungen 30, 31, 32 eingesetzt werden, wobei die Lagerzapfen 18, 19 des Probenbehälters 14 in die Lagermulden 33, 34 der jeweiligen Ausnehmung eingelegt werden, so daß eine kippbare Lagerung des Probenbehälters 14 in der jeweiligen Ausnehmung 30 bzw. 31 bzw. 32 entsteht.

Diametral entgegengesetzt zu den Ausnehmungen 30, 31, 32, die für die Aufnahme des Probenbehälters 14 bestimmt sind. sind auf der gegenüberliegenden Seite der Lagersäule 24 Rastmulden 37, 38, 39 in die Grundplatte 23 eingelassen. Die Rastmulden sind geeignet, den unteren Teil des Exzenters 28 aufzunehmen.

Durch Schwenken des Hebels 26 kann der Meßfühler 27 über dem in einer der Ausnehmungen 30, 31, 32 eingesetzten Probenbehälter 14 positioniert und auf dessen Ringwand 15 abgesenkt werden. Da der Meßfühler 27 eine ringförmige elastische Dichtung 40 aufweist und der Probenbehälter 14 in der Ausnehmung 30 bzw. 31 bzw. 32 infolge entsprechender Anordnung der Lagermulden 33, 34 um eine Achse senkrecht zur Längsachse des Hebels 26 kippbar ist, wird ein hermetischer Abschluß des Innenraumes 16 des Probenbehälters 14 gegenüber der Außenluft erreicht, wenn der Meßfühler 27 durch Senkrechtstellen des Betätigungshebels 29 aufgrund der Exzenterwirkung des Exzenters 28 gegen den Probenbehälter 14 gedrückt wird.

Da in der Meßvorrichtung 22 mehrere Ausnehmungen 30, 31, 32 vorgesehen sind, können Probenbehälter 14 aus mehreren Stanzvorrichtungen 4 gleichzeitig in die Meßvorrichtung 22 eingesetzt werden, so daß der Meßfühler 27 durch horizontales Verschwenken kurzfristig nacheinander Proben aus unterschiedlichen Probenbehältern 14 ausmessen kann. Es ist auch möglich, eine der Ausnehmungen 30, 31, 32 mit einem dem Probenbehälter 14 entsprechenden Behälter auszustatten, in dem eine gesättigte Lösung mit einem Eichsalz vorhanden ist, um die Meßanordnung auf Standardwerte zu eichen.

Der Meßfühler 27 weist eine an sich bekannte Bauart auf und ist deshalb in den Zeichnungen nicht näher dargestellt. In der Ausführungsform gemäß Fig. 4 ist der Feuchte-Meßfühler 27 zusätzlich mit einem nicht näher dargestellten Temperatur-Meßfühler ausgestattet. Über zwei Meßleitungen 41, 42 sind die Meßfühler an eine Anzeigevorrichtung 43 angeschlossen. Sie umfaßt ein Anzeigefeld 44 zur Anzeige der im Meßraum 16 herrschenden relativen Luftfeuchtigkeit und ein Anzeigefeld 45 zur Anzeige der dort herrschenden Temperatur. Im übrigen ist der schaltungsmäßige Aufbau der Meß-und Anzeigevorrichtung bekannt und deshalb in den Zeichnungen nicht dargestellt.

Die Probenbehälter 14 können mit einem nicht dargestellten Deckel verschlossen werden, falls zwischen dem Einbringen der Materialproben in den Probenbehälter und der Durchführung der Feuchtemessung in der Meßvorrichtung 22 eine längerer Zeitraum verstreicht. Die Probenbehälter 14 können auch mit zusätzlichen Einrichtungen zum Konstanthalten der Temperatur im Innenraum 16 versehen werden, falls die Temperatur im Innenraum 16 aufgrund besonderer Raumbedingungen ohne Thermostatisierung größeren Schwankungen unterliegt. Der Feuchte-Meßfühler 27 kann in den Behälter 14 auch fest eingesetzt sein.

Zur Gewinnung der Materialproben 20 aus der Unterlage A unter Bildung der Schnittflächen 21 können anstelle der in Fig. 2 dargestellten Stanzvorrichtung 4 auch andere Vorrichtungen an sich bekannter Bauart verwendet werden, z.B. ein Reißwolf. Die Materialproben 20 können nämlich anstelle der Scheibchenform auch eine Streifenform oder andere Formen aufweisen.

Das erfindungsgemäße Verfahren und die erfindungsgemäße Vorrichtung eignen sich nicht nur für die Bestimmung der Feuchte im Rohpapier von Polyethylen-kaschierten · Unterlagen für fotografische Papiere, sondern auch zur Bestimmung der Feuchte in anderen hygroskopischen flächenhaften Materialien, deren Oberflächen durch entsprechende Beschichtung gegen rasches Eindringen von Feuchtigkeit aus Bädern und dergleichen geschützt sind.

Hierzu zwei Blatt Zeichnungen.


**Ansprüche**

1. Verfahren zur Messung der Gleichgewichtsfeuchte in hygroskopischem, flächenhaftem, beidseitig beschichtetem Material, vorzugsweise in beidseitig beschichtetem Rohpapier der Unterlage eines fotografischen Papiers, durch Messung der relativen Feuchte der mit dem Material in Berührung stehenden Luft, dadurch gekennzeichnet, daß Proben dem Material unter Bildung von Schnittflächen entnommen und in einen Behälter eingebracht werden, der gegenüber der Außenluft abschließbar ist, und daß nach Einstellung der Gleichgewichtsfeuchte in diesem Behälter die relative Feuchte der darin enthaltenen Luft gemessen wird.

2. Verfahren nach Anspruch 1, dadurch gekennzeichnet, daß die Schnittflächen aufweisenden Materialproben mittels einer Stanz-oder Schneidvorrichtung aus dem flächenhaften Material gewonnen werden.

3. Verfahren nach einem der vorangehenden Ansprüche, dadurch gekennzeichnet, daß die Temperatur im Behälter konstant gehalten wird.

4. Vorrichtung zur Durchführung des Verfahrens nach einem der vorangehenden Ansprüche, dadurch gekennzeichnet, daß sie eine Stanz-oder Schneidvorrichtung (4) zur Herstellung der Materialproben (20) sowie einen Behälter (14) zu deren Aufnahme umfaßt, wobei dem Behälter (14) ein Feuchte-Meßfühler (27) zugeordnet ist.

5. Vorrichtung nach Anspruch 4, dadurch gekennzeichnet, daß der Meßfühler (27) an den Behälter (14) ansetzbar ist.

6. Vorrichtung nach Anspruch 5, dadurch gekennzeichnet, daß der Meßfühler an einem - schwenkbaren Arm (26) angeordnet ist, in dessen Schwenkbereich mehrere Aufnahmen (30, 31, 32) für Probenbehälter (14) zum wechselweisen Ansetzen des Meßfühlers (27) vorgesehen sind.

7. Vorrichtung nach einem der Ansprüche 4 bis 6, dadurch gekennzeichnet, daß der Feuchte-Meßfühler (27) mit einem Temperatur-Meßfühler kombiniert ist.

8. Vorrichtung nach einem der Ansprüche 4 bis 7, dadurch gekennzeichnet, daß der Behälter eine Einrichtung zum Konstanthalten der Temperatur aufweist.

FIG. 1

FIG.2

FIG.3

FIG.4